(19) Europäisches Patentamt
European Patent Office
Office européen des brevets

(11) EP 0 950 053 B1

(12) **FASCICULE DE BREVET EUROPEEN**

(45) Date de publication et mention de la délivrance du brevet:
**11.06.2003 Bulletin 2003/24**

(21) Numéro de dépôt: **97953948.3**

(22) Date de dépôt: **24.12.1997**

(51) Int Cl.[7]: **C07D 211/70**, A61K 31/44

(86) Numéro de dépôt international:
**PCT/FR97/02424**

(87) Numéro de publication internationale:
**WO 98/028274 (02.07.1998 Gazette 1998/26)**

# (54) NOUVEAUX DERIVES DE BENZOYLALKYL-1,2,3,6-TETRAHYDROPYRIDINES

NEUE DERIVATE VON BENZOYLALKYL-1,2,3,6-TETRAHYDROPYRIDINEN

NEW DERIVATIVES OF BENZOYLALKYL-1,2,3,6-TETRAHYDROPYRIDINS

(84) Etats contractants désignés:
**AT BE CH DE DK ES FI FR GB GR IE IT LI LU MC NL PT SE**

(30) Priorité: **24.12.1996 FR 9615957**

(43) Date de publication de la demande:
**20.10.1999 Bulletin 1999/42**

(73) Titulaire: **SANOFI-SYNTHELABO
75013 Paris (FR)**

(72) Inventeurs:
- **BARONI, Marco
I-20010 Vanzago (IT)**
- **CARDAMONE, Rosanna
I-22100 Como (IT)**
- **FOURNIER, Jacqueline
F-31830 Plaisance du Touch (FR)**
- **GUZZI, Umberto
I-20148 Milan (IT)**
- **IELMINI, Alessandra
I-21010 Arsago Seprio (IT)**

(56) Documents cités:
**WO-A-91/08200** **WO-A-93/11107**

- **D.I. SCHUSTER ET AL: J. MED. CHEM., vol. 36, no. 24, 1993, pages 3923-3928, XP002040460**
- **M. RAJSNER ET AL: COLLECTION CZECHOSLOV. CHEM. COMMUN., vol. 28, 1963, pages 1031-1043, XP002040461**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 950 053 B1

## Description

La présente invention concerne des nouveaux dérivés de benzoylalkyl-1,2,3,6-tétrahydropyridines, un procédé pour leur préparation et des compositions pharmaceutiques les contenant.

EP-0 458 696 décrit l'utilisation d'une 1-(2-naphtyléthyl)-4-(3-trifluorométhylphényl)-1,2,3,6-tétrahydropyridine pour la préparation de médicaments destinés au traitement de troubles cérébraux et neuronaux.

WO 91/08200 décrit des dérivés de la tétrahydropyridine à activité protectrice vis-à-vis des dommages causés par les états hypoxiques/ischémiques.

WO 93/11107 décrit certaines cétones utilisées comme intermédiaires dans la préparation des alcools correspondant.

D.I Schuster et al., J. Med. Chem. 1993, 36, 3923-3928 décrivent une étude de réceptologie d'une série de N-(1-arylpropionyl)-4-aryl-1,2,3,6-tétrahydropyridines vis-à-vis des récepteurs σ, notamment les récepteurs σ cérébraux ainsi que leur utilisation potentielle comme antipsychotiques.

Il a été maintenant trouvé que certaines benzoylalkyl-1,2,3,6-tétrahydropyridines exercent une action neurotrophique sur le système nerveux semblable à celle du facteur de croissance nerveuse (NGF de l'anglais Nerve Growth Factor) et peuvent rétablir le fonctionnement des cellules endommagées ou présentant des anomalies dans leurs fonctions physiologiques.

La présente invention concerne donc, selon un de ses aspects, l'utilisation des composés de formule(I)

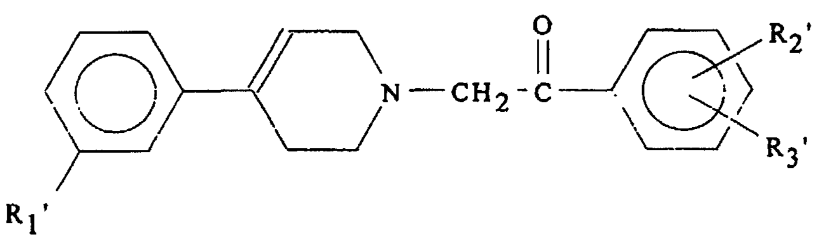

I')

dans laquelle

- $R_{1'}$ représente un halogène, un groupe $CF_3$, $(C_1$-$C_4)$alkyle ou $(C_1$-$C_4)$alcoxyle;
- $R_{2'}$ représente un $(C_1$-$C_6)$alkyle; un $(C_1$-$C_6)$alcoxyle; un halogène; un groupe $CF_3$; un hydroxy, ou un groupe choisi parmi un $(C_3$-$C_7)$cycloalkyle, un phényle, phénoxy, phénylméthyle, phényléthyle, ledit groupe pouvant être mono- ou polysubstitué sur le groupe phényle par un halogène, $CF_3$, $(C_1$-$C_4)$alkyle, $(C_1$- $C_4)$alcoxy;
- $R_{3'}$ représente l'hydrogène, un $(C_1$-$C_6)$alkyle; un $(C_1$-$C_6)$alcoxyle; un halogène; un groupe $CF_3$; un hydroxy;

ainsi que de leurs sels et solvates et de leurs sels d'ammonium quaternaire, sont des composés nouveaux et constituent un aspect ultérieur de la présente invention. Les sels d'ammonium quaternaire préférés sont ceux de formule (I')

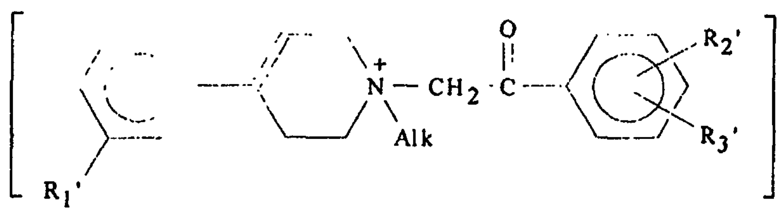

$. X^-$ (I ')

où $X^-$ est un anion pharmaceutiquement acceptable, Alk étant de préférence méthyle et $X^-$ étant de préférence $Cl^-$,$Br^-$, $I^-$, $CH_3SO_3^-$, $C_6H_5SO_3^-$.

Parmi les composée de formule (I) des composés particulièrement avantageux sont les suivant

- la 1-[2-(3'-chloro-4-biphénylyl)-2-oxo-éthyl]-4-(3-trifluorométhyl-phényl)-1,2,3,6-tétrahydropyridine;
- la 1-[2-(2'-chloro-4-biphénylyl)-2-oxo-éthyl]-4-(3-trifluorométhyl-phényl)-1,2,3,6-tétrahydropyridine;
- la 1-[2-(4'-chloro-4-biphénylyl)-2-oxo-éthyl]-4-(3-trifluorométhyl-phényl)-1,2,3,6-tétrahydropyridine;
- la 1-[2-(4-isobutylphényl)-2-oxo-éthyl]-4-(3-trifluorométhyl-phényl)-1,2,3,6-tétrahydropyridine;
- la 1-[2-(4-benzylphényl)-2-oxo-éthyl]-4-(3-trifluorométhyl-phényl)-1,2,3,6-tétrahydropyridine;
- la 1-[2-(4-cyclohexylphényl)-2-oxo-éthyl]-4-(3-trifluorométhyl-phényl)-1,2,3,6-tétrahydropyridine;

la 1-[2-(4'-fluoro-4-biphénylyl)-2-oxo-éthyl]-4-(3-trifluorométhyl-phényl)-1,2,3,6-tétrahydropyridine;
la 1-[2-(4-n-butylphényl)-2-oxo-éthyl]-4-(3-trifluorométhyl-phényl)-1,2,3,6-tétrahydropyridine;
la 1-[2-(4biphénylyl)-2-oxo-éthyl]-4-(3-trifluorométhyl-phényl)-1,2,3,6-tétrahydropyridine;
la 1-[2-(4-*t*-butylphényl)-2-oxo-éthyl]-4-(3-trifluorométhyl-phényl)-1,2,3,6-tétrahydropyridine;
la 1-[2-(3,4-diéthylphényl)-2-oxo-éthyl]-4-(3-trifluorométhyl-phényl)-1,2,3,6-tétrahydropyridine;
la 1-[2-(2'-trifluorométhylbiphényl)-2-oxo-éthyl]-4-(3-trifluorométhyl-phényl)-1,2,3,6-tétrahydropyridine;
la 1-[2-(3'-trifluorométhylbiphényl)-2-oxo-éthyl]-4-(3-trifluorométhyl-phényl)-1,2,3,6-tétrahydropyridine;
la 1-[2-(4'-trifluorométhylbiphényl)-2-oxo-éthyl]-4-(3-trifluorométhyl-phényl)-1,2,3,6-tétrahydropyridine;

leurs sels et solvates pharmaceutiquement acceptables.

Selon un autre de ses aspects, la présente invention concerne un procédé pour la préparation des composés de formule (I), de leurs sels ou solvates et de leurs sels d'ammonium quaternaire, caractérisé en ce que

(a) on fait réagir une aryl-1,2,3,6-tétrahydropyridine de formule (II)

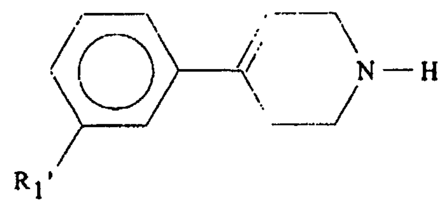

(II)

dans laquelle $R_{1'}$ est tel que défini ci-dessus, avec un composé de formule (III)

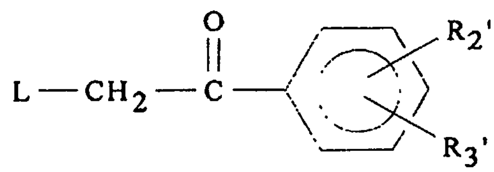

(III)

dans laquelle $R_{2'}$ et $R_{3'}$ sont tels que définis précédemment et L représente un groupe partant, tel que par exemple un atome de chlore, de brome, d'iode, le groupe méthanesulfonyloxy, benzènesulfonyloxy, p-toluènesulfonyloxy, trifuorométhylsulfonyloxy, le brome étant préféré;

(b) on isole le composé de formule (I) ainsi obtenu et, éventuellement on le transforme en l'un de ses sels ou solvates ou l'un de ses sels d'ammonium quaternaire.

La réaction est conduite dans un solvant organique et à une température comprise entre la température ambiante et la température de reflux du solvant utilisé.

Comme solvant organique préférentiel, on utilise un alcool aliphatique ayant de 1 à 6 atomes de carbone, tel que méthanol, éthanol, isopropanol, n-butanol, n-pentanol, mais aussi d'autres solvants tels que hexane, diméthylformamide, diméthylsulfoxyde, sulfolane, acétonitrile, pyridine et similaires peuvent être utilisés.

La réaction est avantageusement conduite en présence d'un agent basique, tel qu'un hydroxyde ou carbonate alcalin ou la triéthylamine, surtout dans le cas où L est un atome d'halogène.

La température de réaction peut varier entre la température ambiante (environ 20°C) et celle de reflux et les temps de réaction varient en conséquence. En général, après 0,5 à 12 heures de chauffage au reflux, la réaction est terminée et le produit final ainsi obtenu peut être isolé selon les techniques conventionnelles sous forme de base libre ou d'un de ses sels ou solvates et la base libre est éventuellement transformée dans un de ses sels par simple salification dans un solvant organique tel qu'un alcool, de préférence l'éthanol ou l'isopropanol, un éther comme le 1,2-diméthoxyéthane, l'acétate d'éthyle, l'acétone ou un hydrocarbure comme l'hexane.

Le composé de formule (I) obtenu, isolé selon ces techniques usuelles, est éventuellement transformé en un de ses sels d'ammonium quaternaire par réaction avec un halogénure d'alkyle de formule (IV)

$$\text{Alk-Hal} \qquad \text{(IV)}$$

où Alk est un $(C_1-C_4)$ alkyle et Hal est chlore, brome, ou iode.

Le sel d'ammonium quaternaire ainsi obtenu de formule (I"a)

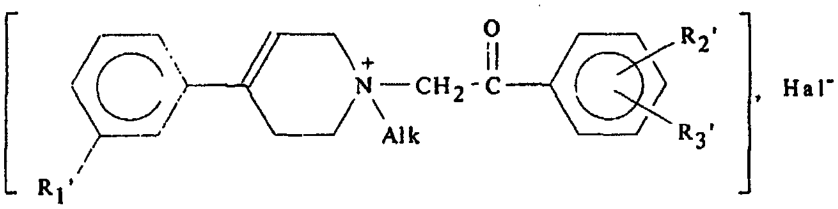

(I")

peut être transformé dans un autre sel par exemple pear une résine échangeuse d'anions chargée $(X')^-$, où X' est un anion pharmaceutiquement acceptable autre que Hal, de préférence l'anion $CH_3SO_3^-$, $C_6H_5SO_3^-$ ou p-$CH_3$-$C_6H_5SO_3^-$ (paratoluènesulfonyloxy).

Lorsque les sels du composé de formule (I) sont préparés pour être administrés en tant que médicaments, il faut que les acides employées soient pharmaceutiquement acceptables; si l'on prépare des sels du composé de formule (I) dans un autre but, par exemple pour mieux purifier le produit ou pour mieux effectuer des essais analytiques ou pour séparer les énantiomères en présence d'un atome de carbone chiral, on peut alors utiliser n'importe quel acide ou base convenable.

Les sels avec des acides pharmaceutiquement acceptables sont par exemple, ceux avec les acides minéraux, tels que le chlorhydrate, le bromhydrate, le borate, le phosphate, le sulfate, l'hydrogénosulfate, l'hydrogénophosphate le dihydrogénophosphate et ceux avec les acides organiques, tels que le citrate, le benzoate, l'ascorbate, le méthylsulfate, le naphtalene-2-sulfonate, le picrate, le fumarate, le maléate, le malonate, l'oxalate, le succinate, l'acétate, le tartrate, le mésylate, le tosylate, l'iséthionate, α-cétoglutarate, l'α-glycérophosphate, le glucose-1-phosphate.

Les amines de départ de formule (II) sont des composés connus ou bien elles peuvent être préparées selon des procédés analogues à ceux utilisés pour préparer les composés connus.

Les composés de formule (III) peuvent être préparés par réaction du benzène approprié de formule (V)

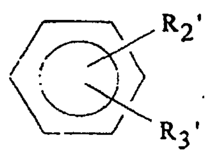

(V)

dans laquelle $R_{2'}$ et $R_{3'}$ sont tels que définis précédemment, avec un halogénure d'acyle de formule L-$CH_2$-CO-Hal en présence d'un acide de Lewis selon la réaction bien connue de Friedel-Crafts.

Alternativement les produits de départ (III) où $R_3$ est un groupe phényle, éventuellement substitué, peuvent également être préparés en effectuant la réaction de Sukuzi en opérant en milieu aqueux., à savoir par condensation entre des phényl dérivés substitués par un groupe partant et acides benzèneboroniques, en présence d'un catalyseur, de bases fortes et d'un agent de transfer de phase selon les conditions décrites par D. Badone et al., 212th ACS National Meeting, American Chemical Society, Orlando FL, August 25-29 1996, Abstract 351).

L'activité des composés de formule (I) sur le système nerveux a été démontrée dans des études *in vitro* et *in vivo* selon les méthodes décrites dans EP-0 458 696 et, pour l'évaluation de la survie neuronale, à l'aide d'un test de survie *in vitro* conduit en utilisant des neurones isolés à partir de dissections de la région septale d'embryons de rats.

Plus particulièrement on a prélevé la région septale d'embryons de rats agés de 17-18 jours sous microscope à dissection dans des conditions stériles, puis on l'a dissociée dans un milieu trypsine-EDTA. La suspension de cellules a été placée dans un flacon de culture dans un milieu DME/Ham's F12 (v:v) (Dulbecco Modified Eagle Medium/ Nutrient Mixture Ham's F12 - R.G. Ham, Proc. Nat. Sci, 1965, 53:288) contenant du sérum de veau à 5% et du sérum de cheval à 5% et maintenue à 37°C pendant 90 minutes. Ce traitement permet l'élimination des cellules non-neuronales.

Les neuroblastes sont ensuite ensemencés dans les puits d'une plaque de titration à raison de $17x10^4$ cellules/$cm^2$, dans un milieu de culture non sérique constitué par du DME/Ham's F12 contenant du sélénium (30 nM) et de la transferrine (1,25 μM). Chaque puits a été préalablement traité à la poly-L-lysine. Les plaques ensemencées sont placées dans un incubateur à l'étuve (37°C; 5% $CO_2$).

Les composés à tester sont dissouts dans du DMSO et dilués comme requis par le milieu de culture.

Les neuroblastes sont maintenus dans des plaques contenant le composé à tester ou le solvant correspondant pendant 4 jours sans changer le milieu.

Après 4 jours le milieu est remplacé par un sel de tétrazolium dissout dans le milieu de culture (0,15 mg/ml).

Les cellules sont ensuite placées à l'étuve à 37°C pendant 4 heures. Les succinodéhydrogénases mitochondriales des cellules vivantes réduisent le sel de tétrazolium en bleu formazan dont, après dissolution dans le DMSO, on mesure la densité optique à 540nm, densité qui est linéairement corrélée au nombre de cellules vivantes (Manthorpe et al., Dev. Brain Res., 1988, 25:191-198).

La différence entre les groupes contenant les composés à tester et les témoins a été évaluée par analyse statistique en utilisant le test t bilatéral de Dunnett ("two-tailed Dunnett t-test").

Dans ce dernier test les composés de formule (I) se sont montrés aussi actifs ou plus actifs que les composés décrits dans EP-0 458 696, l'efficacité de certains composés de formule (I) vis-à-vis de la survie neuronale étant double par rapport au composé A décrit dans EP-0 458 696.

Grâce à cette puissante activité neuroprotectrice, et à leur faible toxicité compatible pour une utilisation en tant que médicaments, les composés de formule (I) ainsi que leurs sels d'addition pharmaceutiquement acceptables, leurs solvates et leurs sels d'ammonium quaternaire, notamment ceux de formule (I'), sont utilisables pour la préparation de compositions pharmaceutiques indiquées dans le traitement et/ou la prophylaxie de toutes les maladies qui impliquent une dégénérescence neuronale, utilisation qui constitue un aspect ultérieur de la présente invention.. Plus particulièrement, l'invention concerne l'utilisation des composés de formule (I), notamment (I'), seuls ou en co-administration ou association avec d'autres principes actifs agissant sur le SNC, par exemple les cholinomimétiques M1 sélectifs, les antagonistes NMDA, les nootropiques tels que le piracétam, dans les indications suivantes: troubles de la mémoire, démence vasculaire, troubles post-encéphalitiques, troubles post-apoplectiques, syndromes post-traumatiques dus à un traumatisme crânien, maladie d'Alzheimer, démence sénile, démence subcorticale, telle que la chorée de Huntington et la maladie de Parkinson, démence provoquée par le SIDA, neuropathies dérivées de morbidité ou dommage des nerfs sympatiques ou sensoriels, et maladies cérébrales, telles que l'oedème cérébral, et les dégénérescences spinocérébelleuses, les dégénérescences des motoneurones, comme par exemple la sclérose latérale amyotrophique.

L'administration des composés selon l'invention peut être convenablement effectuée par voie orale, parentérale, sublinguale ou transdermique. La quantité de principe actif à administrer dans le traitement des troubles cérébraux et neuronaux selon la méthode de la présente invention dépend de la nature et de la gravité des affections à traiter ainsi que du poids des malades. En général, la dose globale chez l'homme étant variable entre 1 et 1400 mg par jour, avantageusement entre 2 et 900 mg par jour, par exemple de 3 à 500 mg, plus convenablement de 10 à 300 mg par jour dans des compositions pharmaceutiques. Les compositions de la présente invention sont administrées de préférence sous forme d'unités de dosage. Ces doses unitaires comprendront généralement de 0,5 à 700 mg, avantageusement de 2 à 300 mg, de préférence de 5 à 150 mg, par exemple entre 5 et 50 mg, à savoir 1, 2, 5, 10, 15, 20, 25, 30, 40 ou 50 mg, de produit. Ces doses unitaires seront administrées normalement une ou plusieurs fois par jour, par exemple 2, 3, 4, ou 5 fois par jour, de préférence une à trois fois par jour.

Selon un autre de ses aspect, la présente invention a pour objet une composition pharmaceutique contenant, en tant que principes actifs, un composé de formule (I) ci-dessus et un composé indiqué dans le traitement symptomatique de la démence sénile du type Alzheimer (DAT) ou leurs sels pharmaceutiquement acceptables.

L'expression "composé indiqué dans le traitement symptomatique de la démence sénile du type Alzheimer (DAT)" indique un produit qui est capable d'améliorer le cadre symptomatologique des patients atteints par la DAT sans intervenir sur les causes de la maladie.

De tels composés sont par exemple les inhibiteurs de l'acétylcholinestérase, les agonistes muscariniques $M_1$, les agonistes nicotiniques, les antagonistes du récepteur NMDA, les nootropiques.

Des inhibiteurs de l'acétylcholinestérase préférés sont le donépézil et le tacrine.

D'autres inhibiteurs de l'acétylcholinestérase pouvant être utilisés sont par exemple la rivastigmine (SDZ-ENA-713), la galanthamine, le métrifonate, l'eptastigmine, la velnacrine, la physostigmine (Drugs, 1997, 53(5): 752-768; The Merck Index 12 ed.).

D'autres inhibiteurs de l'acétylcholinestérase sont encore la 5,7-dihydro-3-[2-[1-(phénylméthyl)-4-pipéridinyl] éthyl]-6H-pyrrolo[3,2-f]-1,2-benzisoxazol-6-one nommée aussi icopezil (J. Med. Chem., 1995, 38: 2802-2808), le MDL-73,745 ou zifrosilone (Eur. J. Pharmacol., 1995, 276: 93-99), le TAK-147 (J. Med. Chem., 1994, 37: 2292-2299).

D'autres inhibiteurs de l'acétylcholinestérase sont par exemple ceux qui sont décrits dans les demandes de brevet JP 09-095483, WO 97/13754, WO 97/21681, WO 97/19929, ZA 96-04565, US 5,455,245, WO 95-21822, EP 637 586, US 5,401,749, EP 742 207, US 5,547,960, WO 96/20176, WO 96/02524, EP 677 516, JP 07-188177, JP 07-133274, EP 649 846, EP 648 771, JP 07-048370, US 5,391,553, WO 94/29272, EP 627 400.

Selon un autre de ses aspects, la présente invention concerne une composition pharmaceutique contenant, en tant que principe actif, un composé de formule (I) et un agoniste du récepteur $M_1$, ou leurs sels pharmaceutiquement acceptables.

Des agonistes du récepteur $M_1$ sont par exemple la milaméline, la bésipiridine, la talsaclidine, la xanoméline, le YM-796 et le YM-954 (Eur. J. Pharmacol., 1990, 187: 479-486), le 3-[N-(2-diéthylamino-2-méthylpropyl)-6-phényl-5-propyl]-pyridazinamine, nommée aussi SR-46559 (Biorg. Med. Chem. Let., 1992, **2:** 833:838), le AF-102, lc CI-979,

le L-689, 660, le LU 25-109, le S-99 77-2, le SB 202,026, la thiopilocarpine, le WAL 2014 (Pharmacol. Toxicol., 1996, 78: 59-68).

Selon un autre de ses aspects, l'invention concerne une composition pharmaceutique contenant, en tant que principe actif, un composé de formule (I) et un agoniste nicotinique ou leurs sels pharmaceutiquement acceptables.

Des agonistes nicotiniques avantageux sont par exemple le MKC-231 (Biorg. Med. Chem. Let., 1995, 5 (14): 1495-1500), le T-588 (Japan J. Pharmacol., 1993, 62: 81-86), le ABT-418 (Br. J. Pharmacol., 1997, 120: 429-438).

Selon un autre de ses aspects, l'invention concerne une composition pharmaceutique contenant, en tant que principe actif, un composé de formule (I) et un antagoniste des récepteurs NMDA ou leurs sels pharmaceutiquement acceptables.

Un antagoniste des récepteurs NMDA avantageux est par exemple la mémantine (Arzneim. Forsch., 1991, 41: 773-780).

Selon un autre de ses aspects, l'invention concerne une composition pharmaceutique contenant, en tant que principe actif un composé de formule (I) et un agent nootropique, ou leurs sels pharmaceutiquement acceptables.

Des agents nootropiques qui peuvent être utilisés selon l'invention sont par exemple le netiracetam, le nebracetam (Merck Index, $12^{th}$ ed.).

Les doses des deux principes actifs associés sont choisies en général parmi les doses qui seraient administrées pour chaque médicament dans le traitement non combiné.

Selon un aspect ultérieur, la présente invention concerne aussi une méthode de traitement de la démence sénile du type Alzheimer qui consiste à administrer à un patient atteint de cette maladie une dose efficace d'un composé de formule (I) ou d'un de ses sels pharmaceutiquement acceptables et une dose efficace d'un composé indiqué dans le traitement symptomatique de la DAT ou d'un de ses sels pharmaceutiquement acceptables, lesdites administrations étant simultanées, séquentielles ou étalées dans le temps et les doses efficaces des principes actifs pouvant être contenues dans des formes d'administration unitaires séparées ou bien, lorsque les principes actifs sont administrés simultanément, les deux principes actifs étant avantageusement contenus dans une forme pharmaceutique unique.

Ainsi, la présente invention concerne, selon un autre de ses aspects, des compositions pharmaceutiques contenant, en tant que principe actif, un composé de formule (I) ou l'un de ses sels ou solvates pharmaceutiquement acceptables ou l'un de ses sels d'ammonium quaternaire, notamment de formule (I').

Dans les compositions pharmaceutiques de la présente invention pour l'administration orale, sublinguale, sous-cutanée, intramusculaire, intraveineuse, transdermique ou rectale, le principe actif peut être administré sous forme unitaire d'administration, soit tel quel par exemple sous forme lyophilisée, soit en mélange avec des supports pharmaceutiques classiques, aux animaux et aux êtres humains pour le traitement des affections susdites. Les formes unitaires d'administration appropriées comprennent les formes par voie orale telles que les comprimés éventuellement sécables, les gélules, les poudres, les granules et les solutions ou suspensions orales, les formes d'administration sublinguale et buccale, les formes d'administration sous-cutanée, intramusculaire ou intraveineuse, les formes d'administration locale et les formes d'administration rectale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

Les exemples qui suivent illustrent mieux l'invention.

EXEMPLE 1

**Chlorhydrate de 1-[2-(3'-chlorobiphényl-4-yl)-2-oxoéthyl]-4-(3-trifluorométhylphényl)-1,2,3,6-tétrahydropyridine.**

**1a/** ***1-bromo-2-(3'-chlorobiphényl-4-yl)éthanone.***

On refroidit à 0-5°C un mélange de 5 g (0,026 mole) de 3-chlorobiphényle, 50 ml de chlorure de méthylène, 6,95 g (0,034 mole) de bromure de bromoacétyle et on y ajoute 4 g (0,030 mole) de trichlorure d'aluminium. On agite pendant 1 heure à 5°C puis 4 heures à la température ambiante. On verse dans un mélange eau/glace, on extrait au chlorure de méthylène, on lave la phase organique avec une solution 1N de HCl, on sèche sur du sulfate de sodium et on évapore sous pression réduite. On obtient 4,5 g du produit du titre. P.f. 63-65°C.

***1b/ Chlorhydrate de 1-[2-(3'-chlorobiphényl-4-yl)-2-oxoéthyl]-4-(3-trifluorométhylphényl)-1,2,3,6-tétrahydropyridine***

On chauffe au reflux pendant 1 heure un mélange de 0,4 g (0,013 mole) du produit de l'étape précédente, 2,95 g (0,013 mole) de 4-(3-trifluorométhylphényl)-1,2,3,6-tétrahydropyridine, 80 ml d'éthanol et 2,32 g (0,0167 mole) de carbonate de potassium anhydre rapé. On élimine les sels par filtration, et on acidifie la solution par addition d'une solution d'éthanol saturée en acide chlorhydrique. On concentre sous pression réduite jusqu'à environ 40 ml et on laisse une nuit à 5°C. On filtre le précipité, on le lave à l'eau et ensuite avec de l'isopropanol. On obtient 4,9 g du composé du titre. P.f. 217-220°C.

EXEMPLE 2

**Chlorhydrate de 1-[2-(2'-chlorobiphényl-4-yl)-2-oxoéthyl]-4-(3-trifluorométhylphényl)-1,2,3,6-tétrahydropyridine.**

En opérant comme décrit dans l'exemple 1 mais en utilisant le 2-chlorobiphényle au lieu du 3-chlorobiphényle, on obtient le composé du titre. P.f. 200-202°C (cristallisé dans l'isopropanol).

EXEMPLE 3

**Chlorhydrate de 1-[2-(4'-chlorobiphényl-4-yl)-2-oxoéthyl]-4-(3-trifluorométhylphényl)-1,2,3,6-tétrahydropyridine.**

En opérant comme décrit dans l'exemple 1 mais en utilisant le 4-chlorpbiphényle au lieu du 3-chlorobiphényle, on obtient le composé du titre. P.f. 210-215°C.

EXEMPLE 4

**Chlorhydrate de 1-[2-(4-isobutylphényl)-2-oxoéthyl]-4-(3-trifluorométhylphényl)-1,2,3,6-tétrahydropyridine.**

En opérant comme décrit dans l'exemple 1 mais en utilisant le 4-isobutylbenzène au lieu du 3-chlorobiphényle, on obtient le composé du titre. P.f. 224-228°C (cristallisé dans l'isopropanol).

EXEMPLE 5

**Chlorhydrate de 1-[2-(4-phénoxyphényl)-2-oxoéthyl]-4-(3-trifluorométhylphényl)-1,2,3,6-tétrahydropyridine.**

En opérant comme décrit dans l'exemple 1 mais en utilisant le diphényléther au lieu du 3-chlorobiphényle, on obtient le composé du titre. P.f. 205-210°C

EXEMPLE 6

**Chlorhydrate de 1-[2-(4-cyclohexylphényl)-2-oxoéthyl]-4-(3-trifluorométhylphényl)-1,2,3,6-tétrahydropyridine.**

En opérant comme décrit dans l'exemple 1 mais en utilisant le cyclohexylbenzène au lieu du 3-chlorobiphényle, on obtient le composé du titre. P.f. 209-213°C (cristallisé dans l'isopropanol).

EXEMPLE 7

**Chlorhydrate de 1-[2-(4'-fluorobiphényl-4-yl)-2-oxoéthyl]-4-(3-trifluorométhylphényl)-1,2,3,6-tétrahydropyridine.**

En opérant comme décrit dans l'exemple 1 mais en utilisant le 4-fluorobiphényle au lieu du 3-chlorobiphényle, on obtient le composé du titre. P.f. 123-125°C (cristallisé dans l'isopropanol).

EXEMPLE 8

**Chlorhydrate de 1-[2-(biphényl-4-yl)-2-oxoéthyl]-4-(3-trifluorométhyl-phényl)-1,2,3,6-tétrahydropyridine.**

En opérant comme décrit dans l'exemple 1 mais en utilisant le biphényle au lieu du 3-chlorobiphényle, on obtient le composé du titre. P.f. 145-147°C (base); P.f. 240-143°C (chlorhydrate).

EXEMPLE 9

**Chlorhydrate de 1-[2-(4-*n*-butylphényl)-2-oxoéthyl]-4-(3-trifluorométhylphényl)-1,2,3,6-tétrahydropyridine.**

En opérant comme décrit dans l'exemple 1 mais en utilisant le 4-*n*-butylbenzène au lieu du 3-chlorobiphényle, on obtient le composé du titre. P.f. 218-221°C.

EXEMPLE 10

**Chlorhydrate de 1-[2-(4-*t*-butylphényl)-2-oxoéthyl]-4-(3-trifluorométhylphényl)-1,2,3,6-tétrahydropyridine.**

En opérant comme décrit dans l'exemple 1 mais en utilisant le 4-*t*-butylbenzène au lieu du 3-chlorobiphényle, on obtient le composé du titre. P.f. 97-9°C (base).

EXEMPLE 11

**Chlorhydrate de 1-[2-(3,4-diéthylphényl)-2-oxoéthyl]-4-(3-trifluorométhylphényl)-1,2,3,6-tétrahydropyridine.**

En opérant comme décrit dans l'exemple 1 mais en utilisant le 3,4-diéthylbenzène au lieu du 3-chlorobiphényle, on obtient le composé du titre. P.f. 232-234°C.

EXEMPLE 12

**Chlorhydrate de 1-[2-(2'-trifluorométhylbiphényl-4-yl)-2-oxoéthyl]-4-(3-trifluorométhylphényl)-1,2,3,6-tétrahydropyridine.**

**12a/** ***2-(4-bromophényl)-2,2-diméthoxyéthane***

On chauffe au reflux pendant 3 heures un mélange de 2 g (0,01 mole) de 4-bromoacétophénone, 5,6 ml d'orthoformiate de triméthyle, 5,6 ml de méthanol et 0,67 g d'Amberlite ® IR 120. Après refroidissement, on filtre sur Célite ® et on évapore la solution filtrée. On obtient 2,4 g du produit du titre sous forme huileuse.

**12b/** ***2,2-diméthoxy-2-(2'-trifluorométhylbiphényl-4-yl)éthane***

On agite à 70°C pendant 1 heure, un mélange de 4,9 g (14 mmole) du produit de l'étape précédente, 2,45 g (16 mmole) d'acide 2-trifluorométhylbenzèneboronique, 63 mg (0,28 mmole) d'acétate de palladium, 4,84 g (35 mmole) de carbonate de potassium et 4,5 g (14 mmole) de bromure de tétrabutylammonium dans 19 ml d'eau. On laisse refroidir et on extrait à l'acétate d'éthyle. On sèche la phase organique sur du sulfate de sodium, on filtre et on évapore le solvant sous pression réduite. On obtient le composé du titre sous forme huileuse.

**12c/** ***4-(2-trifluorophényl)acétophénone***

Aune solution de 4,6 g (0,0105 mole) du produit de l'étape précédente dans 4 ml de chlorure de méthylène, on ajoute à 0°C une solution de 4 ml d'acide trifluoroacétique et 4 ml d'eau. On agite à la température ambiante pendant 2 heures, on verse dans de l'eau, on extrait au chlorure de méthylène. On sèche la phase organique, on filtre et on évapore le solvant sous pression réduite. On purifie le brut par chromatographie sur colonne de gel de silice en éluant avec un mélange cyclohexane/acétate d'éthyle = 9/l. On obtient 1,97 g du produit du titre.

**12d/** *α-**bromo-4-(2-trifluorométhylphényl)acétophénone***

A une solution de 1,97 g (7,5 mmole) du produit de l'étape précédente dans 5,4 ml de méthanol, on ajoute goutte à goutte à la température de 0°C, 0,38 ml (7,5 mmole) de brome. On agite à la température ambiante pendant 3 heures, on évapore le solvant, on reprend le résidu dans de l'eau et on extrait à l'acétate d'éthyle. On sèche la phase organique sur du sulfate de sodium, on filtre et on évapore le solvant sous pression réduite. On obtient le produit du titre.

**12e/** ***Chlorhydrate de 1-[2-(2'-trifluorométhylbiphényl-4-yl)-2-oxoéthyl]-4-(3-trifluorométhylphényl)-1,2,3,6-tétrahydropyridine***

On chauffe au reflux pendant 1 heure un mélange de 0,74 g (0,0028 mole) de 4-(3-trifluorométhylphényl)-1,2,3,6-tétrahydropyridine, 14 ml d'éthanol et 1,27 g (0,0092 mole) de carbonate de potassium anhydre rapé. On y ajoute une solution de 1,2 g (0,0035 mole) de l'huile de l'étape précédente dans 3 ml d'éthanol et on laisse au reflux pendant 30 minutes. On élimine les sels par filtration, et on acidifie la solution par addition d'une solution aqueuse d'acide chlorhydrique 1 N. On évapore le solvant sous pression réduite, on extrait au chloroforme, on sèche la phase organique sur du sulfate de sodium, on filtre et on évapore le solvant sous pression réduite. On libère la base à l'aide d'une solution d'ammoniaque concentrée, on extrait à l'acétate d'éthyle, et on purifie le produit par chromatographie sur colonne de gel de silice en éluant avec un mélange cyclohexane/acétate d'éthyle = 8'2 On obtient le composé du titre. On prépare le chlorhydrate à l'aide d'une solution d'isopropanol saturée en acide chlorhydrique. P.f. 195-197°C.

EXEMPLE 13

**Chlorhydrate de 1-[2-(3'-trifluorométhylbiphényl-4-yl)-2-oxoéthyl]-4-(3-trifluorométhylphényl)-1,2,3,6-tétrahydropyridine.**

En opérant comme décrit dans l'exemple 12 mais en utilisant l'acide 3-trifluorométhylbenzèneboronique au lieu de l'acide 2-trifluorométhylbenzèneboronique dans l'étape 12b/, on obtient le composé du titre. P.f. 232-234°C.

EXEMPLE 14

**Chlorhydrate de 1-[2-(4'-trifluorométhylbiphényl-4-yl)-2-oxoéthyl]-4-(3-trifluorométhylphényl)-1,2,3,6-tétrahydropyridine.**

En opérant comme décrit dans l'exemple 12 mais en utilisant l'acide 4-trifluorométhylbenzèneboronique au lieu de l'acide 2-trifluorométhylbenzèneboronique dans l'étape 12b/, on obtient le composé du titre. P.f. 245-247°C.

## Revendications

**1.** Composés de formule (I)

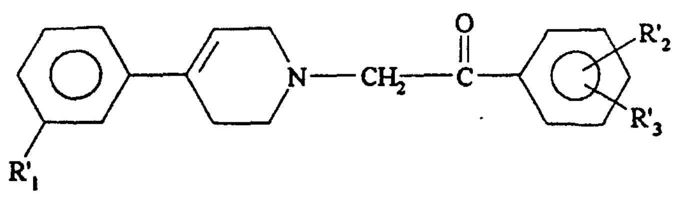

(I)

dans laquelle

$R_{1'}$ représente un halogène, un groupe $CF_3$, $(C_1-C_4)$alkyle ou $(C_1-C_4)$alcoxyle;
$R_{2'}$ représente un $(C_1-C_6)$alkyle; un $(C_1-C_6)$alcoxyle; un halogène; un groupe $CF_3$; un hydroxy, ou un groupe choisi parmi un $(C_3-C_7)$cycloalkyle, un phényle, phénoxy, phénylméthyle, phényléthyle, ledit groupe pouvant être mono- ou polysubstitué sur le groupe phényle par un halogène, $CF_3$, $(C_1-C_4)$alkyle, $(C_1-C_4)$alcoxy;
$R_{3'}$ représente l'hydrogène, un $(C_1-C_6)$alkyle; un $(C_1-C_6)$alcoxyle; un halogène; un groupe $CF_3$; un hydroxy; ainsi que de leurs sels et solvates et de leurs sels d'ammonium quaternaire

**2.** Composé selon la revendication 1 de formule (I')

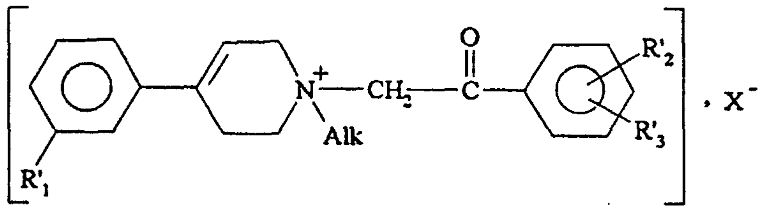

(I')

où $X^-$ est un anion pharmaceutiquement acceptable, Alk est un $(C_1-C_4)$alkyle et $R'_1$, $R'_2$ et $R'_3$ sont tels que définis pour les composés (I) dans la revendication 1.

**3.** Composé selon la revendication 1 choisi parmi

la 1-[2-(3'-chloro-4-biphénylyl)-2-oxo-éthyl]-4-(3-trifluorométhylphényl)-1,2,3,6-tétrahydropyridine;
la 1-[2-(2'-chlorobiphénylyl-4-yl)-2-oxoéthyl]-4-(3-trifluorométhylphényl)-1,2,3,6-tétrahydropyridine;
la 1-[2-(4'-chlorobiphénylyl-4-yl)-2-oxoéthyl]-4-(3-trifluorométhylphényl)-1,2,3,6-tétrahydropyridine;
la 1-[2-(4-isobutylphényl)-2-oxoéthyl]-4-(3-trifluorométhylphényl)-1,2,3,6-tétrahydropyridine;
la 1-[2-(4-benzylphényl)-2-oxoéthyl]-4-(3-trifluorométhylphényl)-1,2,3,6-tétrahydropyridine;
la 1-[2-(4-cyclohexylphényl)-2-oxoéthyl]-4-(3-trifluorométhylphényl)-1,2,3,6-tétrahydropyridine;
la 1-[2-(4'-fluorobiphénylyl-4-yl)-2-oxoéthyl]-4-(3-trifluorométhylphényl)-1,2,3,6-tétrahydropyridine:
la 1-[2-(biphénylyl-4-yl)-2-oxoéthyl]-4-(3-trifluorométhylphényl)-1,2,3,6-tétrahydropyridine;
la 1-[2-(4-*n*-butylphényl)-2-oxo-éthyl]-4-(3-trifluorométhyl-phényl)-1,2,3,6-tétrahydropyridine;
la 1-[2-(4-*n*-butylphényl)-2-oxoéthyl]-4-(3-trifluorométhylphényl)-1,2,3,6-tétrahydropyridine;
la 1-[2-(4-*t*-butylphényl)-2-oxoéthyl]-4-(3-trifluorométhylphényl)-1,2,3,6-tétrahydropyridine;
la 1-[2-(3,4-diéthylphényl)-2-oxoéthyl]-4-(3-trifluorométhylphényl)-1,2,3,6-tétrahydropyridine;
la 1-[2-(2'-trifluorométhylbiphényl-4-yl)-2-oxoéthyl]-4-(3-trifluorométhylphényl)-1,2,3,6-tétrahydropyridine;
la 1-[2-(3'-trifluorométhylbiphényl-4-yl)-2-oxoéthyl]-4-(3-trifluorométhylphényl)-1,2,3,6-tétrahydropyridine;
la 1-[2-(4'-trifluorométhylbiphényl-4-yl)-2-oxoéthyl]-4-(3-trifluorométhylphényl)-1,2,3,6-tétrahydropyridine;
leurs sels et solvates.

**4.** Procédé pour la préparation des composés de formule (I) selon la revendication 1, de leurs sels ou solvates et de leurs sels d'ammonium quaternaire, **caractérisé en ce que**

(a) on fait réagir une aryl-1,2,3,6-tétrahydropyridine de formule (II)

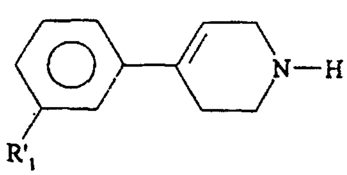

(II)

dans laquelle $R'_1$ est tel que défini pour les composés (I) dans la revendication 1, avec un composé de formule (III)

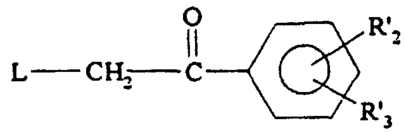

(III)

dans laquelle $R'_2$ et $R'_3$ sont tels que définis pour les composés (I) dans la revendication 1 et L représente un groupe partant; et

(b) on isole le composé de formule (I) ainsi obtenu et, éventuellement on le transforme en l'un de ses sels ou solvates ou l'un de ses sels d'ammonium quaternaire

5. Composition pharmaceutique contenant en tant que principe actif un composé selon l'une des revendications 1 à 3.

6. Composition pharmaceutique contenant en tant que principe actif un composé de formule I selon la revendication 1 ou un composé de formule I' selon la revendication 2 et un composé indiqué dans le traitement symptomatique de la démence sénile du type Alzheimer (DAT), choisi parmi les inhibiteurs de l'acétylcholinestérase, les agonistes muscariniques M1, les agonistes nicotiniques, les antagonistes du récepteur NMDA et les nootropiques, et leurs sels pharmaceutiquement acceptables.

7. Utilisation d'un composé selon l'une des revendications 1 à 3 pour la préparation de médicaments indiquées dans le traitement et/ou la prophylaxie des troubles de la mémoire, de la démence vasculaire, des troubles post-traumatiques dûs à un traumatisme crânien, de la maladie d'Alzheimer, de la démence sénile, de la démence subcorticale telle que la chorée de Huntington et la maladie de Parkinson, de la démence provoquée par le SIDA, des neuropathies dérivées de morbidité ou dommage des nerfs.

**Patentansprüche**

1. Verbindungen der Formel (I)

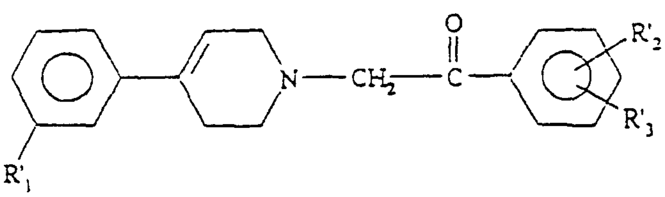

(I)

in der

- $R'_1$ ein Halogen, eine $CF_3$-, ($C_1$-$C_4$)-Alkyl- oder ($C_1$-$C_4$)-Alkoxygruppe darstellt;
- $R'_2$ eine ($C_1$-$C_6$)-Alkylgruppe; eine ($C_1$-$C_6$)-Alkoxygruppe; ein Halogen; eine $CF_3$-Gruppe; eine Hydroxygruppe oder eine Gruppe ausgewählt aus ($C_3$-$C_7$)-Cy-cloalkyl, Phenyl, Phenoxy, Phenylmethyl und Phenylethyl, wobei die Gruppe an der Phenylgruppe durch Halogen, $CF_3$, ($C_1$-$C_4$)-Alkyl oder ($C_1$-$C_4$)-Alkoxy mono- oder polysubstituiert sein kann;
- $R'_3$ Wasserstoff, eine ($C_1$-$C_6$)-Alkylgruppe; eine ($C_1$-$C_6$)-Alkoxygruppe; ein Halogen; eine $CF_3$-Gruppe; oder eine Hydroxy bedeuten;

sowie deren Salze und Solvate und deren quartäre Ammoniumsalze.

2. Verbindung nach Anspruch 1 der Formel (I')

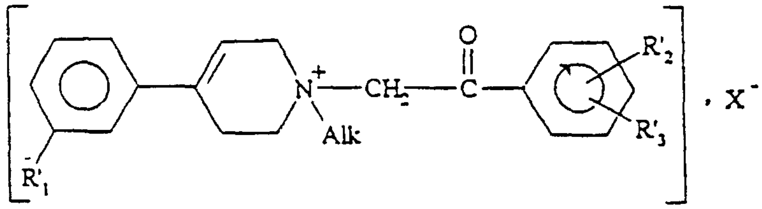

(I')

in der $X^-$ ein pharmazeutisch annehmbares Anion und Alk eine ($C_1$-$C_4$)-Alkylgruppe bedeuten und $R'_1$, $R'_2$ und $R'_3$ die bezüglich der Verbindungen (I) in Anspruch 1 angegebenen Bedeutungen besitzen.

**3.** Verbindung nach Anspruch 1 ausgewählt aus

1-[2-(3'-Chlor-4-biphenylyl)-2-oxo-ethyl]-4-(3-trifluormethylphenyl)-1,2,3,6-tetrahydropyridin:
1-[2-(2'-Chlorbiphenylyl-4-yl)-2-oxoethyl]-4-(3-trifluormethylphenyl)-1,2,3,6-tetrahydropyridin;
1-[2-(4'-Chlorbiphenylyl-4-yl)-2-oxoethyl]-4-(3-trifluormethylphenyl)-1,2,3,6-tetrahydropyridin:
1-[2-(4-Isobutylphenyl)-2-oxoethyl]-4-(3-trifluormethylphenyl)-1,2,3,6-tetrahydropyridin;
1-[2-(4-Benzylphenyl)-2-oxoethyl]-4-(3-trifluormethylphenyl)-1,2,3,6-tetrahydropyridin;
1-[2-(4-Cyclohexylphenyl)-2-oxoethyl]-4-(3-trifluormethylphenyl)-1,2,3,6-tetrahydropyridin;
1-[2-(4'-Fluorbiphenylyl-4-yl)-2-oxoethyl]-4-(3-trifluormethylphenyl)-1,2,3,6-tetrahydropyridin;
1-[2-(Biphenylyl-4-yl)-2-oxoethyl]-4-(3-trifluormethylphenyl)-1,2,3,6-tetrahydropyridin;
1-[2-(4-*n*-Butylphenyl)-2-oxo-ethyl]-4-(3-trifluormethylphenyl)-1,2,3,6-tetrahydropyridin;
1-[2-(4-*n*-Butylphenyl)-2-oxoethyl]-4-(3-trifluormethylphenyl)-1,2,3, 6-tetrahydropyridin;
1-[2-(4-*tert*.-Butylphenyl)-2-oxoethyl]-4-(3-trifluormethylphenyl)-1,2,3,6-tetrahydropyridin;
1-[2-(3,4-Diethylphenyl)-2-oxoethyl]-4-(3-trifluormethylphenyl)-1,2,3,6-tetrahydropyridin;
1-[2-(2'Trifluormethylbiphenyl-4-yl]-2-oxoethyl]-4-(3-trifluormethylphenyl)-1,2,3,6-tetrahydropyridin;
1-[2-(3'-Trifluormethylbiphenyl-4-yl)-2-oxoethyl]-4-(3-trifluormethylphenyl)-1,2,3,6-tetrahydropyridin;
1-[2-(4'-Trifluormethylbiphenyl-4-yl)-2-oxoethyl]-4-(3-trifluormethylphenyl)-1,2,3,6-tetrahydropyridin;

deren Salze und deren Solvate.

**4.** Verfahren zur Herstellung der Verbindungen der Formel (I) nach Anspruch 1, von deren Salzen oder Solvaten und deren quaternären Ammoniumsalzen, **dadurch gekennzeichnet, daß** man

(a) ein Aryl-1,2,3,6-tetrahydropyridin der Formel (II)

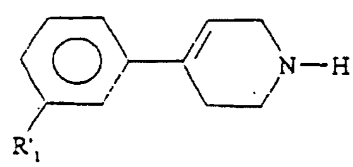

(II)

in der $R'_1$ die für die Verbindungen (I) in Anspruch 1 angegebenen Bedeutungen besitzt, mit einer Verbindung der Formel (III)

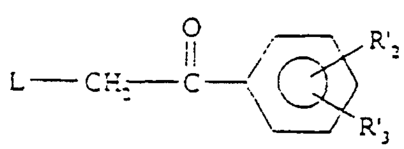

(III)

in der $R'_2$ und $R'_3$ die für die Verbindungen (I) in Anspruch 1 angegebenen Bedeutungen besitzen und L eine austretende Gruppe darstellt, umsetzt; und
(b) die in dieser Weise erhaltene Verbindung der Formel (I) isoliert und gegebenenfalls in eines ihrer Salze

oder Solvate oder eines ihrer quaternären Ammoniumsalze umwandelt.

**5.** Pharmazeutische Zubereitung enthaltend als Wirkstoff eine Verbindung nach einem der Ansprüche 1 bis 3.

**6.** Pharmazeutische Zubereitung enthaltend als Wirkstoff eine verbindung der Formel I nach Anspruch 1 oder eine Verbindung der Formel I' nach Anspruch 2 und eine bei der symptomatischen Behandlung der senilen Demenz vom Typ Alzheimer (DAT) indizierte Verbindung ausgewählt aus Inhibitoren der Acetylcholinesterase, Muscarinagonisten M1, Nicotinagonisten, Antagonisten des NMDA-Rezeptors und nootrope Mittel und deren pharmazeutisch annehmbare Salze.

**7.** Verwendung einer Verbindung nach einem der Ansprüche 1 bis 3 für die Herstellung von Arzneimitteln, die für die Behandlung und/oder die Prophylaxe von Gedächtnisstörungen, der vaskulären Demenz, post-traumatischen Störungen als Folge eines Schädeltraumas, der Azheimerschen Krankheit, der senilen Demenz, der subcorticalen Demenz, wie der Huntington-Chorea und der Parkinsonschen Krankheit, der durch AIDS hervorgerufenen Demenz und von Neuropathien. die durch die Morbidität oder Nervenschädigungen hervorgerufen sind, indiziert sind.

## Claims

**1.** Compounds of formula (I)

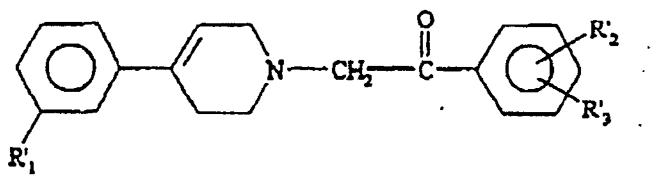

(I)

in which

R'$_1$ represents a halogen or a group $CF_3$, ($C_1$-$C_4$)alkyl or ($C_1$-$C_4$)alkoxy;
R'$_2$ represents a ($C_1$-$C_6$)alkyl; a ($C_1$-$C_6$) alkoxy; a halogen; a $CF_3$ group; a hydroxyl or a group chosen from a ($C_3$-$C_7$)cycloalkyl, a phenyl, phenoxy, phenylmethyl and phenylethyl, the said group possibly being mono- or polysubstituted on the phenyl group with a halogen, $CF_3$, ($C_1$-$C_4$)alkyl or ($C_1$-$C_4$)alkoxy;
R'$_3$ represents hydrogen, a ($C_1$-$C_6$)alkyl; a ($C_1$-$C_6$)alkoxy; a halogen; a $CF_3$ group; a hydroxyl;

and also the salts and solvates thereof and the quaternary ammonium salts thereof.

**2.** Compound according to Claim 1 of formula (I')

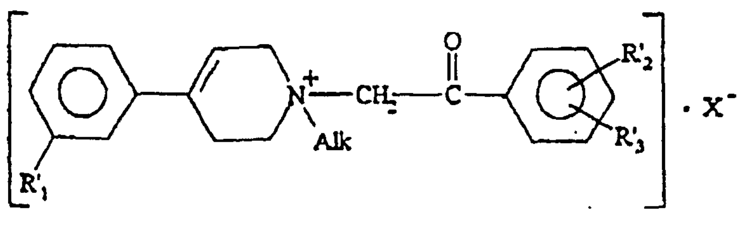

(I')

in which $X^-$ is a pharmaceutically acceptable anion, Alk is a ($C_1$-$C_4$)alkyl and R'$_1$, R'$_2$ and R'$_3$ are as defined for the compounds (I) in Claim 1.

**3.** Compound according to Claim 1, chosen from

1-[2-(3'-chloro-4-biphenylyl)-2-oxoethyl]-4-(3-trifluoromethylphenyl)-1,2,3,6-tetrahydropyridine;
1-[2-(2'-chlorobiphenylyl-4-yl)-2-oxoethyl]-4-(3-trifluoromethylphenyl)-1,2,3,6-tetrahydropyridine;
1-[2-(4'-chlorobiphenylyl-4-yl)-2-oxoethyl]-4-(3-trifluoromethylphenyl)-1,2,3,6-tetrahydropyridine;
1-[2-(4-isobutylphenyl)-2-oxoethyl]-4-(3-trifluoromethylphenyl)-1,2,3,6-tetrahydropyridine;
1-[2-(4-benzylphenyl)-2-oxoethyl]-4-(3-trifluoromethylphenyl)-1,2,3,6-tetrahydropyridine;
1-[2-(4-cyclohexylphenyl)-2-oxoethyl]-4-(3-trifluoromethylphenyl)-1,2,3,6-tetrahydropyridine;
1-[2-(4'-fluorobiphenylyl-4-yl)-2-oxoethyl]-4-(3-trifluoromethylphenyl)-1,2,3,6-tetrahydropyridine;
1-[2-(biphenylyl-4-yl)-2-oxoethyl]-4-(3-trifluoromethylphenyl)-1,2,3,6-tetrahydropyridine;
1-[2-(4-*n*-butylphenyl)-2-oxoethyl]-4-(3-trifluoromethylphenyl)-1,2,3,6-tetrahydropyridine;
1-[2-(4-n-butylphenyl)-2-oxoethyl]-4-(3-trifluoromethylphenyl)-1,2,3,6-tetrahydropyridine;
1-[2-(4-t-butylphenyl)-2-oxoethyl]-4-(3-trifluoromethylphenyl)-1,2,3,6-tetrahydropyridine;
1-[2-(3,4-diethylphenyl)-2-oxoethyl]-4-(3-trifluoromethylphenyl)-1,2,3,6-tetrahydropyridine;
1-[2-(2'-trifluoromethylbiphenyl-4-yl)-2-oxoethyl]-4-(3-trifluoromethylphenyl)-1,2,3,6-tetrahydropyridine;
1-[2-(3'-trifluoromethylbiphenyl-4-yl)-2-oxoethyl]-4-(3-trifluoromethylphenyl)-1,2,3,6-tetrahydropyridine;
1-[2-(4'-trifluoromethylbiphenyl-4-yl)-2-oxoethyl]-4-(3-trifluoromethylphenyl)-1,2,3,6-tetrahydropyridine;

and the salts and solvates thereof.

4. Process for preparing the compounds of formula (I) according to Claim 1, the salts or solvates thereof and the quaternary ammonium salts thereof, **characterized in that**

(a) an aryl-1,2,3,6-tetrahydropyridine of formula (II)

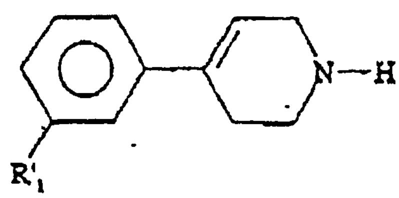

(II)

in which $R'_1$ is as defined for the compounds (I) in Claim 1, is reacted with a compound of formula (III)

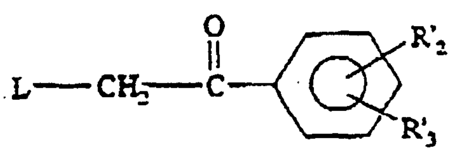

(III)

in which $R'_2$ and $R'_3$ are as defined for the compounds (I) in Claim 1 and L represents a leaving group; and

(b) the compound of formula (I) thus obtained is isolated and optionally converted into a salt or solvate thereof or a quaternary ammonium salt thereof.

5. Pharmaceutical composition containing as active principle a compound according to one of Claims 1 to 3.

6. Pharmaceutical composition containing as active principle a compound of formula I according to Claim 1 or a compound of formula I' according to Claim 2 and a compound indicated in the symptomatic treatment of senile dementia of Alzheimer type (DAT), chosen from acetylcholinesterase inhibitors, muscarine M1 agonists, nicotinic agonists, NMDA receptor antagonists and nootropic agents, and the pharmaceutically acceptable salts thereof.

7. Use of a compound according to one of Claims 1 to 3 for the preparation of medicinal products indicated in the treatment and/or prophylaxis of memory disorders, vascular dementia, post-traumatic disorders caused by a cranial trauma, Alzheimer's disease, senile dementia, subcortical dementia such as Huntington's chorea and Parkinson's disease, AIDS-induced dementia, and neuropathies resulting from nerve morbidity or damage.